# EUROPEAN PATENT SPECIFICATION

(11) **EP 0 828 478 B1**
(45) Date of publication and mention of the grant of the patent: **07.08.2002**
(21) Application number: 96920562.4
(22) Date of filing: 29.05.1996
(51) Int. Cl.: A61K 9/00, A61K 9/48

(54) **OSMOTIC DEVICE WITH DELAYED ACTIVATION OF DRUG DELIVERY AND COMPLETE DRUG RELEASE**
OSMOTISCHE VORRICHTUNG MIT VERZÖGERTEN AKTIVIEREN DER WIRKSTOFFFREISETZUNG UND VOLLSTÄNDIGE ARZNEISTOFFFREISETZUNG
DISPOSITIF OSMOTIQUE AVEC ACTIVATION RETARDEE DE L'APPORT D'UN MEDICAMENT ET LIBERATION COMPLETE DU MEDICAMENT

(30) Priority: 02.06.1995 US 459387; 18.04.1996 US 15567
(43) Date of publication of application: 18.03.1998
(73) Proprietor: ALZA Corporation, Mountain View, CA 94039-7210 (US)
(72) Inventor: DONG, Liang, C., Sunnyvale, CA 94086 (US); WONG, Patrick, S.-L., Palo Alto, CA 94306 (US); YUM, Si-Hong, Daly City, CA 94015 (US); HAMEL, Lawrence, G., Mountain View, CA 94040 (US); DEALEY, Michael, H., San Francisco, CA 94117 (US); POLLOCK, Brenda, J., Mountain View, CA 94043 (US)
(74) Representative: Stebbing, Peter John Hunter
(86) International application number: US9607933
(87) International publication number: WO9638130

(56) References cited:
- US-A- 5 223 265

## Description

### FIELD OF THE INVENTION

The present invention is related to the delayed delivery of an active agent followed by essentially complete delivery of the active agent. More particularly, it is related to osmotically-activated devices for essentially complete dispensing of active agents to a biological environment of use following an initial period of delay.

### BACKGROUND OF THE INVENTION

Oral delivery of therapeutically active agents is a convenient and cost effective method of delivery. The active agent can be released in the mouth or anywhere in the alimentary canal. The delivery can be in a bolus, it can be intermittent, or it can be continuous.

Osmotic dispensing devices for delivery of therapeutically active agents are well known in the art. Such devices use an expansion means to deliver an agent to an environment of use over a period of hours, days or months. The expansion means absorbs liquid, expands, and acts to drive out the beneficial agent formulation from the interior of the device in a controlled, usually constant manner. The osmotic expansion means is used to controllably, usually relatively slowly, and over a period of time, deliver the agent.

Osmotic devices have also been described for prolonged and controlled delivery of one or more active agents where an initial delay of delivery is desired. US Patent No. 5,198,229 is directed to an osmotic device for delivery of an active agent to the upper gastrointestinal tract. The dispensing device comprises concentric housings that are in slidably telescoping arrangement with each other. A first expansion means imbibes fluid when placed in the stomach environment. This expansion means expands and pushes against a partition layer that in turn pushes against an active agent formulation. The active agent is delivered to the stomach environment through a small exit port in a controlled and continuous manner. After all the active agent has been delivered, the housings separate, the buoyancy chamber is exposed to the stomach environment, the density of the device increases, and the device sinks and exits out of the stomach.

US Patent No. 5,312,388 describes the use of slidably telescopic concentric housings in an osmotic device where delivery of more than one active agent is desired or where separate dosings of one active agent is desired. In a particular embodiment, initial rapid delivery of a particular active agent is followed by delayed delivery of the active agent. A loading dose of the active agent is dispensed as soon as the device enters the environment of use. Prolonged delivery is accomplished as a result of an expansion means that imbibes fluid and expands to separate the concentric housings. Upon separation, the active agent contained within the housings is dispensed.

US Patent No. 5,312,390 describes an osmotic device useful for the initially delayed delivery of an active agent. Slidably telescoping concentric housings separate following absorption of fluid through the housing. A fluid passage means is exposed to the fluid environment and the active agent is expelled in a controlled and continuous manner through an exit port at the end of the housing opposite the fluid passage means.

US Patent No. 5,223,265 provides a generally cylindrically shaped delivery device having concentric, slideable housings, which separate upon fluid absorption through one of the housings to provide for an initially delayed release of active agent. Then an expansion member in the housing containing the active agent expands to deliver the active agent through a small opening in the end of the housing over a prolonged period of time.

U.S. Patent No. 5,358,502 describes an osmotic device with a semipermeable membrane with an agent that is pH sensitive and thus will dissolve at a given pH, thereby releasing the contents of the device.

As can be observed in the above-referenced patents, osmotic devices have been described that provide for an initial pulse of an active agent, that provide for prolonged delivery of an active agent, and that provide for delivery of more than one active agent. However, there remains a continuing need for improved methods and systems for delivering one or more active agents in a reliable and reproducible manner.

### SUMMARY OF THE INVENTION

We have observed that devices such as those described above will open in a predictable manner but that the agent contained in the device may not always be completely released to the environment of use following the desired delay period. Accordingly, the present invention is directed to a fluid-imbibing dispensing device and a method for the essentially complete delivery of an active agent to a fluid environment of use following an initially delayed period of delivery of the agent.

In one aspect, the invention is directed to a fluid-imbibing delivery device formed of a first housing and a second housing. The housings are in reversibly sliding telescoping arrangement with each other. The first housing contains an active agent delivery chamber with an active agent formulation and a first expansion agent. The active agent formulation comprises at least one active agent. An open end in the first housing provides for delivery of the active agent formulation to the environment of use. The second housing contains an expansion chamber for separating apart the first and second housings of the device after exposure to the environment of use. The expansion chamber comprises a second expansion agent and optionally a piston. The first expansion agent within the first housing ensures essentially complete release of the active agent formulation to the environment of use.

In another aspect, the invention is directed to a fluid imbibing delivery device formed of a first housing and a closure for the housing. The housing contains an active agent formulation and a first expansion agent. The active agent formulation comprises at least one active agent. The housing is configured to provide a flow path between the interior of the housing and the active agent formulation. The closure comprises a second expansion agent.

The invention is further directed to a device for dispensing an active agent formulation to an environment of use following an initial period of delay, that comprises a housing with a closed end and an open end. The housing contains an active agent formulation that comprises at least one active agent. The housing further contains an expansion agent. The device further comprises a plug sealing the open end of the housing. The improvement of the invention comprises a fluid flow path between the interior of the housing and the active agent formulation.

The invention is also directed to a device for dispensing an active agent formulation to an environment of use following an initial period of delay, that comprises a first impermeable housing and a second semipermeable housing. The first and second housings are in reversibly sliding telescoping arrangement with each other. The first housing contains an active agent formulation that comprises at least one active agent. The first housing further contains a first expansion agent. The second housing contains a second expansion agent. The first housing is configured to provide a flow path between the interior of the first housing and the active agent formulation.

### DESCRIPTION OF THE DRAWINGS

The drawings are not drawn to scale, but are set forth to illustrate various embodiments of the invention. Like numbers refer to like structures.

FIG. 1 is a side cross-sectional view of one embodiment of the delivery device of the present invention, the device being in closed or prepared form prior to placement in the environment of use.

FIG. 2 shows the device of FIG. 1 in operation after placement in the environment of use, showing the second expansion means expanded and the first and second housings of the device separated to allow activation of the first expansion means to begin delivery of the active agent formulation to the environment.

FIG. 3 shows the first housing of the device of FIG. 1 in operation at the end of its useful life, with the first expansion means expanded and the active agent formulation delivered to the environment.

FIG. 4 is a side cross-sectional view of a further embodiment of the delivery device of the present invention, the device being in closed or prepared form prior to placement in the environment of use.

FIG. 5 is a side cross-sectional view of yet another embodiment of the delivery device of the present invention, the device being in closed or prepared form prior to placement in the environment of use.

FIG. 6 is a side cross-sectional view of yet another embodiment of the device of the invention in closed form prior to placement in the environment of use.

FIG. 7 is a side cross-sectional view of still another embodiment of the device of the invention in closed form prior to placement in the environment of use.

FIG. 8 is a graph showing the release characteristics of 6 inventive devices.

### DETAILED DESCRIPTION OF THE INVENTION

The present invention provides a device which is useful for delivering an active agent formulation to a fluid environment of use. There is an initial delay period to startup or activation of the device, this delay period lasts a predetermined length of time, and is followed by essentially complete delivery of the active agent formulation to the environment of use.

### DEFINITIONS

The phrase "initial delay period" intends a period of about a few minutes to a period of about a day, preferably between about 1 and 24 hours, and in particular between about 2 and 15 hours and usually in the range of about 2 to 7 hours. The delivery of the agent formulation from the dispensing device, once begun, is continued until essentially all of the active agent formulation is dispensed. By "essentially all of the active agent formulation" is intended at least about 95% of the active agent formulation, preferably above about 97% and usually greater than 98% of the active agent is delivered to the fluid environment of use. The active agent formulation is preferably administered as a bolus, i.e., the dose of active agent in the device is released in a short period of time of one hour or less and usually within about 30 minutes preferably within about 15 minutes of initiation of delivery of the active agent formulation.

By "semipermeable" is intended a material that is permeable to fluid but impermeable to other ingredients contained in the dispensing device and the environment of use. By "impermeable" is meant that the material is impermeable to fluids as well as to other ingredients contained in the dispensing device such that the migration of such materials into or out of the device is so low as to have substantially no adverse impact on the function of the device during the delivery period.

The term "active agent formulation" intends the active agent optionally in combination with pharmaceutically acceptable carriers and additional inert ingredients. It is to be understood that more than one active agent may be incorporated into the active agent formulation in a device of this invention, and that the use of the term "agent" or "drug" in no way excludes the use of two or more such agents or drugs. The agents can be in various forms, such as uncharged molecules, components of molecular complexes or nonirritating, pharmacologically acceptable salts. Also, simple derivatives of the agents (such as ethers, esters, amides, etc.) which are easily hydrolyzed by body pH, enzymes, etc., can be employed.

The term "active agent" used herein refers to an agent, drug, compound, composition of matter or mixture thereof which provides some biological, often beneficial, effect. This includes pesticides, herbicides, germicides, biocides, algicides, rodenticides, fungicides, insecticides, antioxidants, plant growth promoters, plant growth inhibitors, preservatives, antipreservatives, disinfectants, sterilization agents, catalysts, chemical reactants, fermentation agents, foods, food supplements, nutrients, cosmetics, drugs, vitamins, sex sterilants, fertility inhibitors, fertility promoters, microorganism attenuators and other agents that benefit the environment of use. As used herein, the terms further include any physiologically or pharmacologically active substance that produces a localized or systemic effect or effects in animals, including warm blooded mammals, humans and primates; avians; domestic household or farm animals such as cats, dogs, sheep, goats, cattle, horses and pigs; laboratory animals such as mice, rats and guinea pigs; fish; reptiles; zoo and wild animals; and the like. The active agents that can be delivered includes inorganic and organic compounds, including, without limitation, drugs which act on the peripheral nerves, adrenergic receptors, cholinergic receptors, the skeletal muscles, the cardiovascular system, smooth muscles, the blood circulatory system, synoptic sites, neuroeffector junctional sites, endocrine and hormone systems, the immunological system, the reproductive system, the skeletal system, autacoid systems, the alimentary and excretory systems, the histamine system and the central nervous system. Suitable active agents may be selected from, for example, proteins, enzymes, hormones, polynucleotides, nucleoproteins, polysaccharides, glycoproteins, lipoproteins, polypeptides, steroids, hypnotics and sedatives, psychic energizers, tranquilizers, anticonvulsants, muscle relaxants, antiparkinson agents, analgesics, anti-inflammatories, local anesthetics, muscle contractants, antimicrobials, antimalarials, hormonal agents including contraceptives, sympathomimetics, polypeptides and proteins capable of eliciting physiological effects, diuretics, lipid regulating agents, antiandrogenic agents, antiparasitics, neoplastics, antineoplastics, hypoglycemics, nutritional agents and supplements, growth supplements, fats, ophthalmics, antienteritis agents, electrolytes and diagnostic agents.

Examples of beneficial active agents useful in this invention include prochlorperazine edisylate, ferrous sulfate, aminocaproic acid, mecaxylamine hydrochloride, procainamide hydrochloride, amphetamine sulfate, methamphetamine hydrochloride, benzphetamine hydrochloride, isoproteronol sulfate, phenmetrazine hydrochloride, bethanechol chloride, methacholine chloride, pilocarpine hydrochloride, atropine sulfate, scopolamine bromide, isopropamide iodide, tridihexethyl chloride, phenformin hydrochloride, methylphenidate hydrochloride, theophylline cholinate, cephalexin hydrochloride, diphenidol, meclizine hydrochloride, prochlorperazine maleate, phenoxybenzamine, thiethylperazine maleate, anisindione, diphenadione erythrityl tetranitrate, digoxin, isoflurophate, acetazolamide, methazolamide, bendroflumethiazide, chlorpropamide, tolazamide, chlormadinone acetate, phenaglycodol, allopurinol, aluminum aspirin, methotrexate, acetyl sulfisoxazole, hydrocortisone, hydrocorticosterone acetate, cortisone acetate, dexamethasone and its derivatives such as betamethasone, triamcinolone, methyltestosterone, 17-β-estradiol, ethinyl estradiol, ethinyl estradiol 3-methyl ether, prednisolone, 17-β-hydroxyprogesterone acetate, 19-nor-progesterone, norgestrel, norethindrone, norethisterone, norethiederone, progesterone, norgesterone, norethynodrel, aspirin, indomethacin, naproxen, fenoprofen, sulindac, indoprofen, nitroglycerin, isosorbide dinitrate, propranolol, timolol, atenolol, alprenolol, cimetidine, clonidine, imipramine, levodopa, chlorpromazine, methyldopa, dihydroxyphenylalanine, theophylline, calcium gluconate, ketoprofen, ibuprofen, cephalexin, erythromycin, haloperidol, zomepirac, ferrous lactate, vincamine, phenoxybenzamine, diltiazem, milrinone, captropril, mandol, quanbenz, hydrochlorothiazide, ranitidine, flurbiprofen, fenbufen, fluprofen, tolmetin, alclofenac, mefenamic, flufenamic, difuninal, nimodipine, nitrendipine, nisoldipine, nicardipine, felodipine, lidoflazine, tiapamil, gallopamil, amlodipine, mioflazine, lisinopril, enalapril, captopril, ramipril, enalaprilat, famotidine, nizatidine, sucralfate, etintidine, tetratolol, minoxidil, chlordiazepoxide, diazepam, amitriptylin, and imipramine. Further examples are proteins and peptides which include, but are not limited to, insulin, colchicine, glucagon, thyroid stimulating hormone, parathyroid and pituitary hormones, calcitonin, renin, prolactin, corticotrophin, thyrotropic hormone, follicle stimulating hormone, chorionic gonadotropin, gonadotropin releasing hormone, bovine somatotropin, porcine somatropin, oxytocin, vasopressin, prolactin, somatostatin, lypressin, pancreozymin, luteinizing hormone, LHRH, interferons, interleukins, growth hormones such as human growth hormone, bovine growth hormone and porcine growth hormone, fertility inhibitors such as the prostaglandins, fertility promoters, growth factors, and human pancreas hormone releasing factor.

As used herein, the terms "therapeutically effective" amount or rate refer to the amount or rate of the active agent needed to effect the desired therapeutic, often beneficial, result.

The dispensing devices of the invention find use, for example, in humans or other animals. The environment of use is a fluid environment and can comprise the stomach, the intestinal tract, or a body cavity such as the peritoneum or vagina. A single dispensing device or several dispensing devices can be administered to a subject during a therapeutic program.

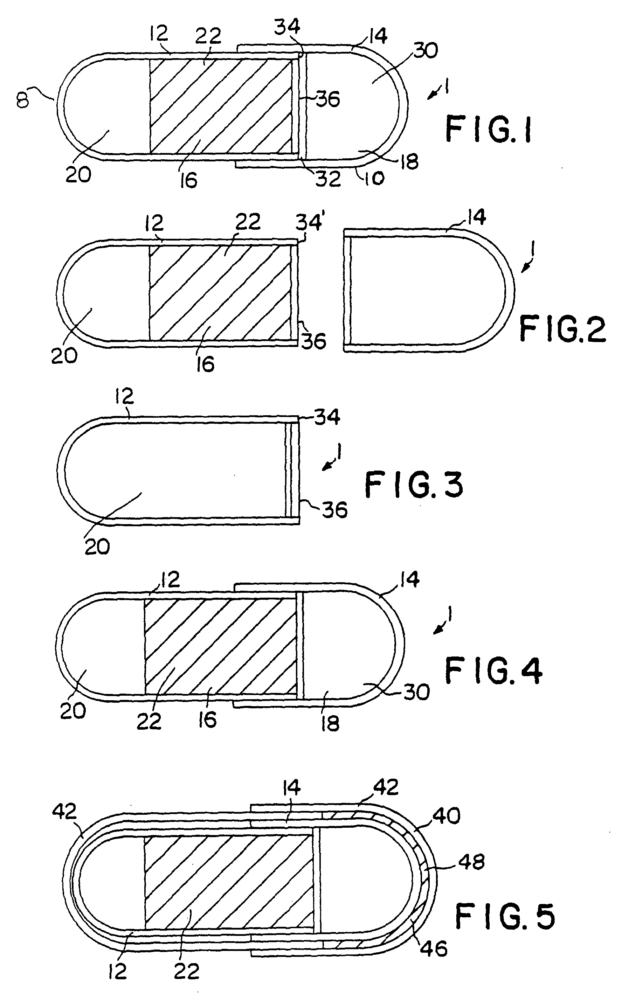

FIG. 1 depicts, in side cross-sectional view, an embodiment of the delivery device according to the present invention. The device is shown in closed or prepared form prior to placement in the environment of use. Dispensing device 1 comprises a housing 8 and a closure 10. Housing 8 and closure 10 in this particular embodiment are shown to be a first impermeable housing 12 and a second semipermeable housing 14. First housing 12 and a second housing 14 are in slidably telescoping arrangement with each other. First housing 12 surrounds and defines an active agent delivery chamber 16 and contains a first expansion agent 20 and an active agent formulation 22.

Second housing 14 encompasses an expansion chamber 18 and contains a second expansion agent 30 and a moveable impermeable partition 32. Partition 32 is positioned between second expansion agent 30 and the open end 36 of first housing 12 which, when exposed to the environment, permits delivery of the active agent formulation to the environment of use.

First housing 12 and second housing 14 at their ends are close in size so that a friction fit is formed between the housings. The friction generated is sufficient to maintain the two housing together prior to activation of the second expansion agent 30 but not so great as to keep the two housings from sliding apart once an expanding driving for is exerted. The end of first housing 12 is adapted to fit within second housing 14. The edge 34 of the end of first housing 12 provides a platform or ridge against which partition 32 abuts to receive the driving force of second expansion agent 30 to separate the two housings.

In operation, dispensing device 1 is placed in the fluid environment of use and second expansion agent 30 begins to imbibe and absorb fluid through second housing 14 from the environment. Second expansion agent 30 expands, exerting a driving force via partition 32 against edge 34 of first housing 12 and the end of formulation 22 to begin to slidably separate first housing 12 from second housing 14. Following separation of the first housing 12 and second housing 14, fluid from the environment of use enters the open end 36 of first housing 12, thus causing first expansion agent 20 to imbibe fluid. As first expansion agent 20 imbibes fluid, it expands and pushes against active agent formulation 22. Agent formulation 22 therefore is expelled from active agent delivery chamber 16 into the environment of use. The expansion agent 20 continues to expand and deliver active agent until the expansion agent 20 reaches the open end 36 of the first housing 12. At that point, which is within an hour, preferably within about 30 minutes and usually within about 15 minutes of the separation of the two housings, essentially all of the active agent formulation 22 has been delivered.

FIG. 2 shows the dispensing device 1 of FIG. 1 in operation after separation of the two housings of the device. First housing 12 has been separated from second housing 14 by the expanding driving force of the second expansion agent 30, which has expanded in size as a result of imbibing fluid from the environment. The open end 36 of the first housing 12 is now exposed to the environment so that the active agent can be delivered.

FIG. 3 shows first housing 12 and the active agent delivery chamber 16 of dispensing device 1 of FIG. 1 after essentially all of the active agent has been delivered to the environment. First expansion agent 20 has expanded in size as a result of imbibing fluid from the open end 36 of first housing 12 to push active agent formulation 22 out of the first housing 12.

FIG.4 shows, in side cross-sectional view, a further embodiment of the delivery device according to the present invention. In this embodiment, the partition 32 shown in FIGS. 1-3 has been omitted and the expansion agent 30 pushes directly against active agent formulation 22 to accomplish separation of housings 12 and 14.

FIG.5 shows, in side cross-sectional view, yet another embodiment of the delivery device according to the present invention. This embodiment is similar to the devices described above. A third housing 40, comprised of a first section 42 and a second section 44, defines a second active agent delivery chamber 46. The active agent formulation 48 contained within the delivery chamber 46 may be the same as the active agent formulation 22 or may be different. The active agent itself may be the same or it may be different. The housing may be comprised of a dissolvable material such as a gelatin capsule so that when introduced into the fluid environment, the housing 40 will dissolve and active agent formulation 48 will be released as a loading dose. Fluid will then be absorbed through housing 14 to effect separation of housing 12 and housing 14 and delivery of active agent formulation 22 as described above with regard to FIG. 1.

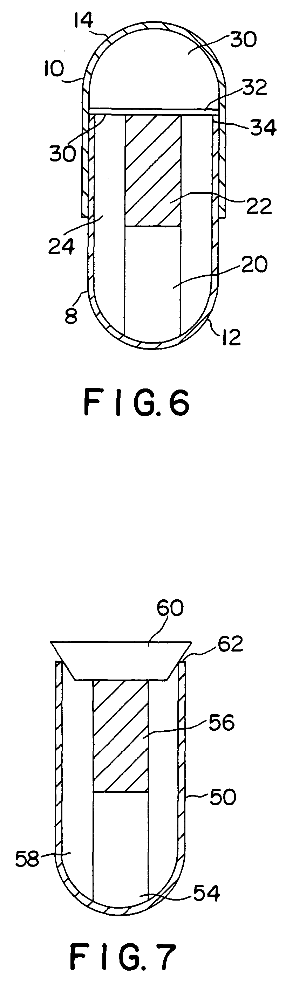

FIG. 6 shows a further embodiment of the delivery device according to the present invention. The device is similar to the device of FIG. 1 in that it comprises a first impermeable housing 12, and a second semipermeable housing 14. The first housing contains a first expansion agent 20 and an active agent formulation 22. In this embodiment, the expansion agent 20 and the active agent formulation 22 do not completely fill the first housing 12. The housing 12 is configured to provide a fluid flow path 24 between the inner wall of housing 12 and the active agent formulation 22 to facilitate rapid fluid entry into housing 12 after separation of the housings and rapid expansion of expansion agent 20 and delivery of active agent formulation 22. FIG. 6 shows the flow path between the inner wall of housing 12 and both the active agent formulation 22 and the first expansion agent 20, but the flow path may be limited to the area between the active agent formulation 22 and the housing 12.

FIG. 7 shows yet another embodiment of the delivery device according to the present invention. In this embodiment a housing 50 contains an expansion agent 54 and an active agent formulation 56. Similar to the device of FIG. 6, the expansion agent 54 and active agent formulation 56 do not completely fill housing 50. Instead, a fluid flow path 58 that facilitates fluid entry into the housing 50 and delivery of the active agent formulation 56 is defined between the interior of housing 50 and both the active agent formulation 56 and the expansion agent 54. FIG. 7 shows the flow path 58 between the inner wall of housing 50 and both the active agent formulation 56 and the expansion agent 54, but the flow path may be limited to the area between the active agent formulation 56 and the housing 50. A second expansion agent in the form of a plug 60 is a closure that seals the open end 62 of housing 50. Upon insertion into the fluid environment of use, the plug 60 swells and separates from the housing 50, thereby permitting fluid entry into the chamber.

Because first expansion agents 20 and 54 operate by imbibing fluid that enters the fluid flow paths 24 and 58 via the open ends 36 and 62 of housings 12 and 50, respectively, the wall of first housings 12 and 50 are preferably comprised of an impermeable material in at least the portion of the housing that is in contact with the first expansion agents 20 and 54. In this way, the first expansion agents 20 and 54 are not prematurely activated. When an active agent or an active agent dosage form is sensitive to fluid from an exterior fluid present in the environment of use, it is preferred that first housings 12 and 50 be substantially impermeable in their entirety to the ingress of the external fluid to serve as a means for substantially protecting the active agent formulations 22 and 56 as well as the first expansion agents 20 and 54.

Because second expansion agent 30 operates by imbibing external fluid while the housings 12 and 14 remain telescopically connected, the wall of second housing 14 in at least the portion that is adjacent to second expansion agent 30 must be semipermeable.

The walls of housings 12, 14 and 50 optionally comprise additional ingredients such as, for example, a plasticizer. Impermeable and semipermeable compositions suitable for use in housings 12, 14, and 50 as well as suitable additives, are known in the art, examples of which are disclosed in U.S. Patent 4,874,388.

The delivery devices of the present invention are nontoxic, biologically inert, nonallergenic and nonirritating to body tissue, and maintain their physical and chemical integrity; that is, the devices do not erode or degrade in the environment of use during the dispensing period. It is within the scope of the invention that the devices be insoluble only during the period of intended use and can thereafter dissolve away in the environment of use. Thus, a dispenser is contemplated which is unaffected by its environment, solubility-wise, at the situs of use or which, alternatively, is only slightly soluble during the period of intended use, such that once its active agent content has been removed it will then dissolve or erode away.

The first and second expansion agents 20, 30, 56 and 60 are nontoxic, nonallergenic and biologically inert. The first and second expansion agents in each device may be the same or they may be different. In one embodiment, the expansion agents comprise an osmopolymer. Osmopolymers interact with water and aqueous biological fluids and swell or expand to an equilibrium state. Osmopolymers exhibit the ability to swell in fluid and to retain a significant portion of the imbibed and absorbed fluid within the polymer structure. The expansion agents in another embodiment comprise an osmagent. Osmagents are also known as osmotically effective solutes and compounds. Osmagents that can be used for the purpose of this invention include inorganic and organic compounds that exhibit an osmotic pressure gradient across a semipermeable, i.e. a fluid-permeable wall. The expansion agents in yet another embodiment comprise an osmagent dispersed within an osmopolymer. The expansion agents can be in tablet or layer form, or can be a plurality of tablets or layers. The osmagent or osmopolymer can be in any suitable form such as particles, crystals, pellets, granules, porous hydrogels, elastic polymeric sponges and the like. Osmagents and osmopolymers are known to the art and are described in, for example, U.S. Pat. Nos. 3,865,108, 4,002,173, 4,207,893, 4,327,725 and 4,612,008.

Partition 32 may comprise a composition that is substantially impermeable to the passage of fluid and that restricts passage of fluid present in the expansion agent into the first housing. It operates to essentially maintain the integrity of the active agent formulation 22 and the expansion layer. Additionally, partition 32 insures that the expanding driving force generated by the second expansion agent 30 is applied directly against first housing 12 to separate the first and second housings. Thus, partition 32 must be of sufficient strength, thickness and rigidity to transfer the driving force against first housing 12. Representative impermeable materials useful as piston 32 are known to the art and described in, for example, U.S. Patent No. 4,874,388.

The active agent formulation comprises the active agent to be delivered, as a liquid, solid, semisolid or thermosensitive composition, generally in a carrier substance and with or without additional inert ingredients. The active agent formulation may additionally include dosage forms comprising the active agent that are capable of maintaining their physical configuration and chemical integrity while housed within the dispenser. These include, without limitation, tablets with or without a density element; matrix tablets; spheres; pellets and elongated tablets; capsules; elementary osmotic pumps, such as those described in U.S. Patent No. 3,845,770; mini-osmotic pumps, such as those described in U.S. Patent Nos. 3,995,631, 4,034,756 and 4,111,202; and multichamber osmotic systems referred to as push-pull and push-melt osmotic pumps, such as those described in U.S. Patent Nos. 4,320,759 and 4,449,983.

The pharmaceutically acceptable carrier may comprise more than one ingredient, such as, for example, a buffer, a viscosity regulating vehicle, a surfactant, dyes, a permeation enhancer, proteinase inhibitors, or other formulation ingredients and additives, as are known in the art. The carrier may contain more than one active agent. The active agent formulation can erode or disintegrate and can be in the form of a wax formulation, solid core or tablet, for example. The formulation can immediately dissolve upon exposure to fluid or it may erode slowly with or without the presence of excipients for controlling erosion.

The active agent formulation can be designed in a multitude of ways to provide a specific drug delivery profile. One embodiment may comprise a formulation that contains a biologically acceptable solid surfactant which is capable of slow dispersion in the environmental fluid. In another embodiment, the formulation may contain a fluid-insoluble wax and a surfactant so that the formulation is susceptible to erosion in the environment. In still another embodiment, the formulation may be effervescent and provide drug delivery in a finely dispersed form. This is accomplished by the addition of a solid basic compound capable of evolving carbon dioxide in the presence of an acid in the environment of use. Suitable basic compounds are disclosed in U.S. Pat. No. 4,265. In a further embodiment, the formulation may include an osmotic agent or solute, such as those described above with reference to the expansion means, so that when the formulation comes into contact with the environmental fluid, it immediately dissolves. In yet another embodiment, the agent formulation can be comprised of an agent and a thermoresponsive composition. In this manner, the formulation would exhibit solid-like properties at room temperature of 21°C and within a few degrees Celsius thereof, and would have a melting point that approximates mammalian body temperatures of 37°C and within a few degrees Celsius thereof. The term "thermoresponsive" as used herein denotes the physical-chemical property of an agent carrier composition to exhibit solid, or solid-like properties at temperatures up to 31°C and become fluid, semi-solid or viscous when disturbed by heat at temperatures from 31°C, usually in the range of 31°C to 45°C. Suitable materials useful as active agent carriers and excipients are known in the art and are disclosed, for example, in U.S. Patent Nos. 4,595,583 and 4,874,388.

The amount of active agent employed in the delivery device will be that amount necessary to deliver a therapeutically effective amount of the agent to achieve the desired result at the site of delivery. In practice, this will vary widely depending upon the particular agent, the site of delivery, the severity of the condition, and the desired therapeutic effect, but will be between 0.01 ng and 500 mg.

For proper delivery of the active agent, it may be desirable in some instances for the dispensing device to deliver active agent to a particular environment of use. Thus, it may be necessary for the device to remain in a particular environment of use until such time as the agent formulation has been delivered or, alternatively, for the device to pass through one particular environment to another prior to delivering agent formulation. In such cases, additional elements are included in the device, or the device is designed in such a way to provide for such particular delivery. For example, when the environment of use is the rumen of a ruminant animal, a density element may be included in the dispensing device so that the device is weighted to remain within the rumen during the dispensing period. Density elements are known in the art and are discussed in, for example, US Patent No. 4,874,388.
When the environment of use is the human stomach, it may be desirable for the device to, for example, have a low initial density or to include air in that portion of the internal compartment of the device that also contains the agent formulation. In this manner, the device will float on the surface of the stomach contents and remain in the stomach until the device opens to release the formulation. Where it is desirable, on the other hand, to delay the release of an active agent which, for example, is inactivated by the stomach contents or may cause nausea or bleeding by irritating the gastric mucosa so that delivery in the stomach is not desired, an enteric coating can be applied over at least that portion of the housing of the dispensing device that is comprised of a semipermeable membrane. Enteric coatings will remain intact in the stomach but will rapidly dissolve once they arrive at the small intestine, thereafter allowing fluid to be imbibed to activate the dispensing device. Enteric coatings are well known in the art and are discussed in, for example, "Remington's Pharmaceutical Sciences", Mack Publishing Co., Easton, PA.

The total delay time prior to separation of the two housings of the dispensing device and the total delivery time of the active agent formulation can be controlled by a number of means to provide a sharp start-up of delivery at a particular time with high accuracy. For example, the rate of fluid imbibition into each of the expansion agent, and thus the rate of expansion of the means, can be controlled by the particular choice of semipermeable membrane or microporous screen. The rate of expansion of the expansion agent can also be controlled by the choice of composition of the expansion agent. The distance of overlap between the telescoping portions of the first and second housings can determine the period of time required for the two housings to separate. Combinations of such control means may be used. Such control means are known in the art and can be determined without undue experimentation.

The fluid flow path 24 or 58 is formed between the active agent formulation 22 and the housings 12 or 50 and may extend to the area between the first expansion agents 20 and 54 and the housings 12 and 50. The space occupied by the fluid flow path is at least 5% of the interior cross-sectional area of the housing, usually between about 5% and 75% of the interior cross-sectional area and often between 20 and 60% of the interior cross-sectional area.

The delivery device of the present invention can be manufactured by standard manufacturing techniques. For example, in the preparation of devices of the present invention, first housing 12 (the vessel) and second housing 14 (the cap) may be separately molded or extruded to the desired shape. Possible semipermeable materials from which the second housing 14 may be prepared include, for example, Hytrel® polyester elastomers (Du Pont), cellulose esters, water flux enhanced ethylene-vinyl acetate copolymers, semipermeable membranes made by blending a rigid polymer with water-soluble low molecular weight compounds, and other semipermeable materials known to the art. Impermeable materials from which the first housing 12 may be prepared include, for example, polyethylene, polystyrene, ethylene-vinyl acetate copolymers, Hytrel® polyester elastomers (Du Pont) and other impermeable materials known to the art. Alternatively, the two portions of a hard gelatin capsule may be coated, one with an impermeable material and the other with a semipermeable material such as cellulose ester-based polymer mixtures. In a presently preferred embodiment, the assembled device in closed configuration is about the size and dimensions of a size "3" to size "OO" hard gelatin capsule. In a presently preferred embodiment, the assembled device in closed configuration is about the size and dimensions of a size "3" to size "OO" hard gelatin capsule.

Expansion agent 20 is prepared from an osmotic material and formed into a shape that will fit within vessel 12. The layer is compressed into a tablet on a rotary bilayer tablet press. Expansion agent 30 is prepared from an osmotic material and partition 32 is prepared from an impermeable material. Both are formed into a shape that will fit within cap 14, and compressed on a bilayer rotary tablet press.

With reference to FIG. 1, the device is assembled as follows. Expansion agent 20 is inserted into the vessel 12 at its end opposite its open end 36. Active agent formulation 22 is then placed on top of expansion agent 20. The formulation may be in the from of a liquid, solid, semi-solid, powder or shaped tablet or tablets, for example. The expansion agent 30 and partition 32 are placed within the cap 14 and the cap assembly is placed over the end of the filled vessel 12 so that partition 32 is adjacent to the open end of the filled vessel 12.

The following examples are illustrative of the present invention. They are not to be construed as a limitation of the scope of the invention.

### EXAMPLE 1

A delivery device according to the present invention for delivering progesterone into the colon for hormone replacement therapy is prepared as follows:

100 mg of Crosspovidone XL-10 (International Specialty Products, Wayne NJ) powder was compressed in a rotary press into a cylindrical tablet to form the first expanding layer portion of the device. One face of the tablet was convex to conform to the shape of the device, while the other face is flat.

The second expanding layer portion of the device was formed from an osmotic layer and a barrier layer. The osmotic layer was formed as follows. Sodium chloride (NaCI) was sized and screened using a Quardo Mill with a 21 mesh screen at the speed set on maximum. This dry component was added to a granulator bowl of a Glatt fluid bed granulator with other dry components in the following proportions: 58.75 wt% sodium carboxymethyl cellulose (NaCMC), 30 wt% NaCI (prepared as described above), 5.0 wt% hydroxypropylmethyl cellulose E-5 (Aqualon), and 1.0 wt% red ferric oxide. The dry components were thoroughly mixed for 10 minutes. 5.0 wt% HPC-EF (Aqualon, Wilmington, DE) was dissolved in purified water and sprayed onto the dry components until the components were in granular form. Magnesium stearate (0.25 wt%) was then added to the granulation and the granulation was thoroughly mixed for 5 min.

The barrier layer part of the second expanding layer portion of the device was formed as follows. 95 wt% hydroxypropylmethyl cellulose E-5 (Aqualon) and 5.0 wt% stearic acid were sized and screened using a 40 mesh screen. The screened materials were added to a mixing vessel of a Hobart mixer and blended for 10 min. Ethanol was then slowly added while mixing until a wet mass was formed. The wet mass was then screened through a 20 mesh screen and the wet granules were allowed to air dry for 12 hours. After drying, the granules were again passed through a 20 mesh screen.

To form the second expanding layer portion of the device, osmotic layer granules (200 mg) and the barrier layer granules (50 mg) were compressed together in a rotary press into a cylindrical bilayer tablet with the osmotic layer face being convex and the barrier layer face being flat. Tabletting produced a clean, distinct interface between the two layers.

The drug layer portion of the device contained 80 wt% progesterone, 10 wt% Crosspovidone XL-10 (International Specialty Products, Wayne NJ) and 9.5 wt% polyoxyethylene 40 stearate (ICI America International, Wilmington, DE). During preparation, each of the components was screened through a 40 mesh screen and the sized components were added to a mixing vessel in the appropriate proportions. The dry components were mixed thoroughly until a wet mass was formed. The wet mass was then screened through a 20 mesh screen and the granules were oven-dried at 40°C for 24 hours. After drying, the granules were passed through a 20 mesh screen. Magnesium stearate (0.5 wt%) was then added to the granulation and the granulation was mixed thoroughly for 5 min. The drug layer granules (125 mg) comprising 100 mg progesterone were then compressed in a rotary press into a cylindrical tablet with the top and bottom faces being flat.

The first housing (drug vessel), with one closed end and one open end, and composed of ethylene vinyl acetate copolymer (9 wt% vinyl acetate), was prepared by placing the pelletized ethylene vinyl acetate copolymer (EVA) in an extruder with a barrel temperature of 130°C and extruding the material into a mold for the vessel. The EVA was allowed to cool in the mold, after which the finished vessel was removed.

A clear, size O gelatin capsule was used to form the second housing (engine assembly) of the device. The second expanding layer portion of the device was placed into the shorter segment of the capsule, the convex end of the tablet pointing into the closed end of the capsule. Polyvinylpyrrolidone (PVP k29-32, International Specialty Products) was dissolved in methanol and sprayed onto the capsule as a 2 mg subcoating. 75 wt% cellulose acetate 398-10 (Eastman Chemical, Kingsport, NJ) and 25 wt% polyethylene glycol 3350 (Union Carbide, Danbury, CT) were dissolved in an acetone/methanol (80/20 wt/wt) solution to make a 4 wt% solid solution. This solution was sprayed onto the subcoating to form a 70 mg semipermeable membrane. The second housing was dried at 50°C and 50 %RH for 72 hours and then at 50°C and ambient RH for 24 hours to anneal the coating and remove the residue solvents.

To assemble the delivery device, the first expanding layer portion of the device was placed into the first housing with the convex face of the tablet pointing into the closed end of the housing. The drug layer was then placed on top of the first expanding layer. The open end of this first housing was fitted into the open end of the second housing and the two housings were compressed together until the first housing, second expanding layer portion and second housing fit together tightly.

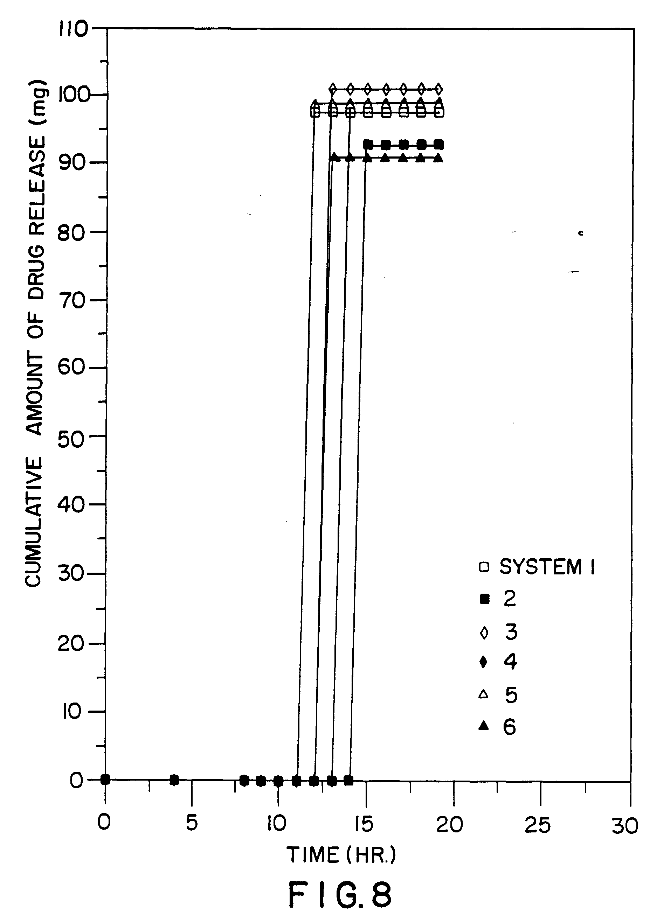

The opening times of the devices are shown in Figure 8. The systems were placed in artificial gastric fluid (pH 1.4) for 2 hours, then, in artificial intestinal fluid for 22 hours. The opening times were determined to be the time interval at which the osmotic caps separated from the drug-containing vessels. The progesterone concentration was measured by HPLC following solubilization with ethanol. Figure 8 shows the progesterone release profiles of six devices, indicated that the opening times were between 11 and 14 hours and the delivery was complete within 1 hour of the opening time.

### EXAMPLE 2

A tablet containing 100 mg progesterone was encased in a #2 size gelatin capsule and administered with the device according to Example 1. The capsule will disintegrate and deliver the initial 100 mg of progesterone immediately upon administration and the remaining 100 mg will be administered 13 hours later.

### EXAMPLE 3

A delivery device according to the present invention for delivering human growth hormone into the colon is prepared as follows:

90 mg of Crosspovidone XL-10 (International Specialty Products) powder was compressed in a rotary press into a cylindrical tablet to form the first expanding layer portion of the device. One face of the tablet was convex to conform to the shape of the device, while the other face is flat.

The second expanding layer portion of the device was formed from an osmotic layer and a barrier layer. The osmotic layer was formed as follows. Sodium chloride (NaCI) was sized and screened using a Quardo Mill with a 21 mesh screen at the speed set on maximum. This dry component was added to a granulator bowl of a Glatt fluid bed granulator with other dry components in the following proportions: 58.75 wt% sodium carboxymethyl cellulose (NaCMC), 30 wt% NaCI (prepared as described above), 5.0 wt% hydroxypropylmethyl cellulose E-5 (Aqualon), and 1.0 wt% red ferric oxide. The dry components were thoroughly mixed for 10 minutes. 5.0 wt% HPC-EF (Aqualon) was dissolved in purified water and sprayed onto the dry components until the components were in granular form. Magnesium stearate (0.25 wt%) was then added to the granulation and the granulation was thoroughly mixed for 5 minutes

The barrier layer part of the second expanding layer portion of the device was formed as follows. 95 wt% hydroxypropylmethyl cellulose E-5 (Aqualon) and 5.0 wt% stearic acid were sized and screened using a 40 mesh screen. The screened materials were added to a mixing vessel of a Hobart mixer and blended for 10 min. Ethanol was then slowly added while mixing until a wet mass was formed. The wet mass was then screened through a 20 mesh screen and the wet granules were allowed to air dry for 12 hours. After drying, the granules were again passed through a 20 mesh screen.

To form the second expanding layer portion of the device, osmotic layer granules (250 mg) and the barrier layer granules (50 mg) were compressed together in a rotary press into a cylindrical bilayer tablet with the osmotic layer face being convex and the barrier layer face being flat. Tabletting produced a clean, distinct interface between the two layers.

The drug layer portion of the device contains 6.67 wt% recombinant human growth hormone (HGH) (ARES-Serono, Norwell, MA), 78.33 wt% sodium salicylate (Bryant Lab, Berkeley, CA) and 15 wt% corn oil (Spectrum Chemical, Gardena, CA). During preparation, the sodium salicylate and HGH were each screened through an 80 mesh screen. The sized components were dry-blended for 15 minutes. The corn oil was added dropwise and mixed until homogeous. The oily mass was then screened through a 40 mesh screen to become granules. The drug layer granules (216 mg) were then compressed in a rotary press into a cylindrical tablet with the top and bottom faces being flat.

The first housing (drug vessel), with one closed end and one open end and composed of ethylene vinyl acetate copolymer (9 wt% vinyl acetate), was prepared by placing the pelletized ethylene vinyl acetate copolymer (EVA) in an extruder with a barrel temperature of 130°C and extruding the material into a mold for the vessel. The EVA is allowed to cool in the mold, after which the finished vessel was removed.

A clear, size O gelatin capsule was used to form the second housing (engine assembly) of the device. The second expanding layer portion of the device was placed into the shorter segment of the capsule, the convex end of the tablet pointing into the closed end of the capsule. 90 wt% cellulose acetate 398-10 (Eastman Chemical, Kingsport, NJ) and 10 wt% polyethylene glycol 3350 (Union Carbide, Danbury, CT) were dissolved in an acetone/methanol (80/20 wt/wt) solution to make a 4 wt% solid solution. This solution was sprayed onto the capsule to form a 29 mg semipermeable membrane. The second housing was dried at 50°C and 50 %RH for 72 hours and then at 50°C and ambient RH for 24 hours to anneal the coating and remove the residue solvents.

To assemble the delivery device, the first expanding layer portion of the device was placed into the first housing with the convex face of the tablet pointing into the closed end of the housing. The drug layer was then placed on top of the first expanding layer. The open end of this first housing was fitted into the open end of the second housing and the two housings were compressed together until the first housing, second expanding layer portion and second housing fit together tightly. The opening time of the system was measured as described in Example 1 and found to be about 6 hours. A dog transit study was conducted in which the devices were fed to dogs. The systems recovered from feces were assayed for the HGH formulation. The results of this study showed that 86% of the HGH formulation on average was delivered as compared to 62% for systems without the first expanding layer portion.

### EXAMPLE 4

Delivery devices for delivering 125 mg of a progesterone formulation into the colon for hormone replacement therapy according to the present invention were prepared as follows.

The first housing, with one closed end and one open end was prepared by placing pelletized ethylene vinyl acetate copolymer (EVA, 9 wt% vinyl acetate) in an extruder with a barrel temperature of 130°C to form a vessel having a 0.762 cm ID and a 0.876 cm OD. An expansion agent tablet was then placed in the first housing. The tablet was prepared from 125 mg of Crosspovidone XL-10 (International Specialty Products, Wayne NJ) powder which had been compressed in a rotary press to have a diameter of 0.556 cm and a length of 0.556 cm. A fluid flow path between the tablet and the inner wall of the housing measured 0.206 cm. An active agent formulation tablet was placed on top of the expansion agent tablet. The 125 mg active agent formulation tablet contained 80 wt% progesterone, 10 wt% Crosspovidone XL-10 (International Specialty Products, Wayne NJ) and 10 wt% polyoxyethylene 40 stearate (ICI America International, Wilmington, DE). The formulation was prepared by screening the components through a 40 mesh screen, mixing with ethanol to form a wet mass, screening through a 20 mesh screen, and oven-drying at 40°C for 24 hours. Magnesium stearate (0.5 wt%) was added. The resultant active agent formulation was compressed in a rotary press into cylindrical tablets having a diameter of 0.556 cm and a length of 0.556 cm. The tablet was placed into the first housing leaving a fluid flow path between the active agent formulation tablet and the inner wall of the first housing of 0.206 cm. The flow path occupied 46% of the interior cross-sectional area of the housing.

The second housing was formed from a clear, size O gelatin capsule. A second expanding layer of the device was formed from the following dry components: 58.75 wt% sodium carboxymethyl cellulose (NaCMC), 30 wt% NaCl, 5.0 wt% hydroxypropylmethyl cellulose E-5 (Aqualon, Wilmington, DE), and 1.0 wt% red ferric oxide. After mixing for 10 minutes, 5.0 wt% HPC-EF (Aqualon, Wilmington, DE), dissolved in purified water was sprayed onto the dry components to prepare granules. Magnesium stearate (0.25 wt%) was added and the second expanding layer granules were thoroughly mixed for 5 minutes. An impermeable partition was formed by screening 95 wt% hydroxypropylmethyl cellulose E-5 (Aqualon) and 5.0 wt% stearic acid using a 40 mesh screen. The screened materials were added to a Hobart mixer and blended for 10 min. Ethanol was added until a wet mass was formed. The wet mass was screened through a 20 mesh screen and allowed to air dry for 12 hours. The granules were then passed through a 20 mesh screen. 200 mg of the expanding layer granules and 50 mg of the partition layer granules were compressed together into a bilayer tablet in a rotary press. The tablet was placed into the gelatin capsule, expanding layer side down. Polyvinylpyrrolidone (PVP k29-32, International Specialty Products) was dissolved in methanol and sprayed onto the capsule as a 2 mg subcoating. 75 wt% cellulose acetate 398-10 (Eastman Chemical, Kingsport, NJ) and 25 wt% polyethylene glycol 3350 (Union Carbide, Danbury, CT) was dissolved in an acetone/methanol (80/20 wt/wt) solution. This 4 wt% solid solution was sprayed onto the subcoating to form a 70 mg semipermeable membrane. The second housing was dried at 50°C and 50 %RH for 72 hours and then at 50°C and ambient RH for 24 hours. The open end of the second housing was fitted over the open end of the first housing and the two housings were compressed together.

### EXAMPLE 5

Eight devices were prepared as described in Example 1. The systems were orally administered to fasted dogs by placing the systems deeply into the oral pharynx of the dogs so that they were swallowed intact. Water was administered immediately following dosing to ensure passage of the system into the stomach.

The dogs were monitored at regular intervals during the normal work day. At each observation, any fecal material that was found in the cages was carefully examined for the presence of the system. The time of recovery of the system was used to estimate the transit time of the device. Table I shows the transit times and amount recovered from these 8 devices.

**TABLE I**

| System # | Transit time (hr.) | Progesterone Residual % |
|---|---|---|
| 1 | 24.5-24.7 | 0.9 |
| 2 | 24.5-24.7 | 0.3 |
| 3 | 24.5-24.7 | 3.9 |
| | | |
| 4 | 48.8-50.5 | 0.0 |
| 5 | not recovered | N/A |
| | | |
| 6 | 26.7-28.2 | 0.0 |
| 7 | 26.7-28.2 | 0.7 |
| 8 | 26.7-28.2 | 0.1 |

As can be seen from Table I, although transit times vary with the individual dogs, the devices delivered greater than 95% of the active agent formulation in a predictable manner. When similar devices were made with no fluid flow path between the housing and the active agent formulation, the delivery was not as predictable since the amount of active agent formulation remaining in the device varied from 0-82%.

## Claims

1. A fluid-imbibing delivery device for dispensing an active agent formulation to a fluid environment of use after an initial, preset delay of startup of delivery, the device comprising:
(a) a first housing and a second housing, the first and second housings being in reversibly sliding telescoping arrangement with each other, the second housing being semipermeable;
(b) said first housing having an open end facing toward and slidably received in the second housing and containing an active agent formulation and a first expansion agent, the active agent formulation located proximal the open end of the first housing and the first expansion agent located distal the open end of the first housing; and
(c) said second housing containing a second expansion agent.

2. The device of claim 1 that further comprises a fluid flow path between the inner wall of the first housing and the active agent formulation.

3. A fluid-imbibing delivery device for dispensing an active agent formulation to a fluid environment of use after an initial, preset delay of startup of delivery, the device comprising a housing and a closure for the housing, the housing containing an active agent formulation comprising at least one active agent proximal the closure and a first expansion agent distal the closure, and being configured to provide a fluid flow path between the inner wall of the housing and the active agent formulation, and the closure comprising a second expansion agent.

4. A fluid imbibing delivery device for dispensing an active agent formulation to a fluid environment of use after an initial, preset delay of startup of delivery, the device comprising a housing having a closed end and an open end, the housing containing an active agent formulation comprising at least one active agent proximal the open end and an expansion agent distal the open end, and a plug sealing the open end of the housing;
and being configured to provide a fluid flow path between the active agent formulation and the inner wall of the housing.

5. The device of claim 4 wherein the plug comprises a second expansion agent.

6. The device of claims 1, 2, 3, or 4 wherein the first housing is impermeable.

7. The device of claims 1, 2, 3 or 5 wherein the first and second expansion agents are selected from the group consisting of osmagents, osmopolymers and mixtures thereof.

8. The device of claims 1, 2, or 3 comprising a partition disposed between said second expansion agent and said active agent formulation.

9. The device of claims 1, 2, 3, or 4 wherein the active agent formulation comprises progesterone.

10. The device of claims 1, 2, 3, or 4 wherein the active agent formulation comprises human growth hormone.

11. The device of claims 1, 2, 3 or 4 wherein the active agent formulation is selected from the group consisting of liquid, solid, semi-solid, thermo-responsive formulations, and mixtures thereof.

12. The device of claims 1, 2, or 3 further comprising a third housing surrounding said first and second housings.

13. The device of claim 12 wherein said third housing defines a second active agent delivery chamber.

14. The device of claims 1, 2, 3, or 4 that further comprises an enteric coating.

## Patentansprüche

1. Flüssigkeitsaufnehmende Abgabevorrichtung zur Abgabe einer Wirkstofformulierung an eine flüssige Einsatzumgebung nach einer anfänglichen, vorgegebenen Verzögerung des Beginns der Abgabe, wobei die Vorrichtung umfaßt:
(a) ein erstes Gehäuse und ein zweites Gehäuse, wobei das erste und das zweite Gehäuse umkehrbar gleitbeweglich und teleskopartig zueinander angeordnet sind, und das zweite Gehäuse semipermeabel ist;
(b) wobei das erste Gehäuse ein offenes Ende hat, das zum zweiten Gehäuse zeigt, und gleitbeweglich in das zweite Gehäuse aufgenommen wird, und eine Wirkstofformulierung und ein erstes Expansionsmittel enthält, wobei sich die Wirkstofformulierung nahe dem offenen Endes des ersten Gehäuses und das erste Expansionsmittel in größerem Abstand vom offenen Ende des ersten Gehäuses befinden; und
(c) wobei das zweite Gehäuse ein zweites Expansionsmittel enthält.

2. Vorrichtung nach Anspruch 1, die weiterhin einen Durchflußweg zwischen der Innenwand des ersten Gehäuses und der Wirkstofformulierung umfaßt.

3. Flüssigkeitsaufnehmende Abgabevorrichtung zur Abgabe einer Wirkstofformulierung an eine flüssige Einsatzumgebung nach einer anfänglichen, vorgegebenen Verzögerung des Beginns der Abgabe, wobei die Vorrichtung ein Gehäuse und einen Verschluß für das Gehäuse umfaßt, und das Gehäuse eine Wirkstofformulierung enthält, welche mindestens einen Wirkstoff nahe dem Verschluß und ein erstes Expansionsmittel in größerem Abstand vom Verschluß umfaßt, und so angeordnet ist, daß ein Durchflußweg zwischen der Innenwand des Gehäuses und der Wirkstofformulierung zur Verfügung gestellt wird, und der Verschluß ein zweites Expansionsmittel umfaßt.

4. Flüssigkeitsaufnehmende Abgabevorrichtung zur Abgabe einer Wirkstofformulierung an eine flüssige Einsatzumgebung nach einer anfänglichen, vorgegebenen Verzögerung des Beginns der Abgabe, wobei die Vorrichtung ein Gehäuse mit einem geschlossenen Ende und einem offenen Ende umfaßt, wobei das Gehäuse eine Wirkstofformulierung enthält, welche mindestens einen Wirkstoff nahe dem offenen Ende und ein Expansionsmittel in größerem Abstand vom offenen Ende umfaßt, sowie einen Stopfen, der das offene Ende des Gehäuses verschließt, und so angeordnet ist, daß ein Durchflußweg zwischen der Wirkstofformulierung und der Innenwand des Gehäuses zur Verfügung gestellt wird.

5. Vorrichtung nach Anspruch 4, bei der der Stopfen ein zweites Expansionsmittel enthält.

6. Vorrichtung nach den Ansprüchen 1, 2, 3 oder 4, bei der das erste Gehäuse undurchlässig ist.

7. Vorrichtung nach den Ansprüchen 1,2,3 oder 5, bei der das erste und das zweite Expansionsmittel aus der Gruppe ausgewählt sind, die aus osmotischen Mitteln, Osmopolymeren und deren Gemischen besteht.

8. Vorrichtung nach den Ansprüchen 1, 2 oder 3, welche eine Trennwand umfaßt, die zwischen dem zweiten Expansionsmittel und der Wirkstofformulierung angeordnet ist.

9. Vorrichtung nach den Ansprüchen 1, 2, 3 oder 4, bei der die Wirkstofformulierung Progesteron umfaßt.

10. Vorrichtung nach den Ansprüchen 1, 2, 3 oder 4, bei der die Wirkstofformulierung menschliches Wachstumshormon umfaßt.

11. Vorrichtung nach den Ansprüchen 1, 2, 3 oder 4, bei der die Wirkstofformulierung aus der Gruppe ausgewählt ist, die aus flüssigen, festen, halbfesten wärmeempfindlichen Formulierungen und deren Gemischen besteht.

12. Vorrichtung nach den Ansprüchen 1, 2 oder 3, welche weiterhin ein drittes Gehäuse umfaßt, welches das erste und zweite Gehäuse umgibt.

13. Vorrichtung nach Anspruch 12, bei der das dritte Gehäuse eine zweite Wirkstoff-Abgabekammer definiert.

14. Vorrichtung nach den Ansprüchen 1, 2, 3 oder 4, die weiterhin eine magensaftresistente Beschichtung umfaßt.

## Revendications

1. Dispositif d'administration par imbibition de fluide, pour distribuer une formulation d'agent actif à un environnement fluide d'utilisation après un retard initial, préétabli, de démarrage de la libération, le dispositif comprenant :
(a) une première enveloppe et une seconde enveloppe, les première et seconde enveloppes étant disposées l'une par rapport à l'autre dans un arrangement télescopique coulissant de manière réversible, la seconde enveloppe étant semiperméable ;
(b) ladite première enveloppe ayant une extrémité ouverte tournée vers et reçue de façon coulissante dans la seconde enveloppe et contenant une formulation d'agent actif et un premier agent d'expansion, la formulation d'agent actif située de façon proximale par rapport à l'extrémité ouverte de la première enveloppe et le premier agent d'expansion étant situé de façon distale par rapport à l'extrémité ouverte de la première enveloppe ; et
(c) ladite seconde enveloppe contenant un second agent d'expansion.

2. Dispositif selon la revendication 1 qui comprend en outre un chemin d'écoulement de fluide entre la paroi interne de la première enveloppe et la formulation d'agent actif.

3. Dispositif d'administration par imbibition de fluide, pour distribuer une formulation d'agent actif à un environnement fluide d'utilisation après un retard initial, préétabli, de démarrage de la libération, le dispositif comprenant une enveloppe et une fermeture pour l'enveloppe, l'enveloppe contenant une formulation d'agent actif comprenant au moins un agent actif situé de façon proximale par rapport à la fermeture et un premier agent d'expansion situé de façon distale par rapport à la fermeture, et étant configuré pour fournir un chemin d'écoulement de fluide entre la paroi interne de l'enveloppe et la formulation d'agent actif, et la fermeture comprenant un second agent d'expansion.

4. Dispositif d'administration par imbibition de fluide, pour distribuer une formulation d'agent actif à un environnement fluide d'utilisation après un retard initial, préétabli, de démarrage de la libération, le dispositif comprenant une enveloppe ayant une extrémité fermée et une extrémité ouverte, l'enveloppe contenant une formulation d'agent actif comprenant au moins un agent actif situé de façon proximale par rapport à l'extrémité ouverte et un agent d'expansion situé de façon distale par rapport à l'extrémité ouverte, et un bouchon scellant l'extrémité ouverte de l'enveloppe ;
et étant configuré pour assurer un chemin d'écoulement de fluide entre la formulation d'agent actif et la paroi interne de l'enveloppe.

5. Dispositif selon la revendication 4, dans lequel le bouchon comprend un second agent d'expansion.

6. Dispositif selon l'une des revendications 1, 2, 3 ou 4, dans lequel la première enveloppe est imperméable.

7. Dispositif selon l'une des revendications 1, 2, 3 ou 5, dans lequel les premier et second agents d'expansion sont choisis dans le groupe constitué par les osmagents, les osmopolymères et leurs mélanges.

8. Dispositif selon l'une des revendications 1, 2 ou 3, comprenant une cloison disposée entre ledit second agent d'expansion et ladite formulation d'agent actif.

9. Dispositif selon l'une des revendications 1, 2, 3 ou 4, dans lequel la formulation d'agent actif comprend de la progestérone.

10. Dispositif selon l'une des revendications 1, 2, 3 ou 4, dans lequel la formulation d'agent actif comprend de l'hormone de croissance humaine.

11. Dispositif selon l'une des revendications 1, 2, 3 ou 4, dans lequel la formulation d'agent actif est choisie dans le groupe constitué par les formulations thermosensibles liquides, solides, semi-solides, et leurs mélanges.

12. Dispositif selon l'une des revendications 1, 2 ou 3, comprenant en outre une troisième enveloppe entourant lesdites première et seconde enveloppes.

13. Dispositif selon la revendication 12, dans lequel ladite troisième enveloppe définit une seconde chambre d'administration d'agent actif.

14. Dispositif selon l'une des revendications 1, 2, 3 ou 4, qui comprend en outre un enrobage entérique.
